# EUROPEAN PATENT SPECIFICATION

(11) **EP 2 272 967 B1**
(45) Date of publication and mention of the grant of the patent: **23.12.2015**
(21) Application number: 10175642.7
(22) Date of filing: 01.10.2004
(51) Int. Cl.: C12N 15/82, A01H 5/00

(54) **Stacking crop improvement traits in transgenic plants**
Sammeln von Kulturpflanzenverbesserungsmerkmalen bei transgenen Pflanzen
Accumulation de traits d'amélioration de plantes agricoles dans des plantes transgéniques

(30) Priority: 02.10.2003 US 508409 P
(43) Date of publication of application: 12.01.2011
(62) Divisional of application: 04793919.4
(73) Proprietor: Monsanto Technology LLC, St. Louis, MO 63167 (US)
(72) Inventor: Armstrong, Charles Lester, O'Fallon, MO 63368 (US); Feng, Paul C.C., Wildwood, MO 63011 (US); Hall, Michael A., Wildwood, MO 63038 (US); Heck, Gregory R., Crystal Lake Park, MO 63131 (US); Stephens, Michael A., Clarkson Valley, MO 63005 (US); Peters, Thomas J., Chesterfield, MO 63005 (US); Post-Beittenmiller, Martha-Ann, St. Louis, MO 53167 (US)
(74) Representative: Von Kreisler Selting Werner - Partnerschaft von Patentanwälten und Rechtsanwälten mbB

(56) References cited:
- WO-A-00/28058
- WO-A-01/70929
- WO-A-02/04650
- WO-A-99/54471
- WO-A-02/063020
- WO-A-03/076612
- FRANCOIS ISABELLE E J A ET AL: "Different approaches for multi-transgene-stacking in plants" PLANT SCIENCE (SHANNON), vol. 163, no. 2, August 2002 (2002-08), pages 281-295, XP002328497 ISSN: 0168-9452
- COLOVA-TSOLOVA V ET AL: "Multiple transgene in grape for seedless and stress tolerance" IN VITRO CELLULAR AND DEVELOPMENTAL BIOLOGY ANIMAL, vol. 38, no. Abstract, April 2002 (2002-04), page 83.A, XP009047708 & 2002 CONGRESS ON IN VITRO BIOLOGY; ORLANDO, FL, USA; JUNE 25-29, 2002 ISSN: 1071-2690

## Description

### Field of the Invention

Disclosed herein are DNA useful for producing transgenic plants.

### Background Of The Invention

One of the goals of plant genetic engineering is to produce plants with agronomically, horticulturally or economically important traits including tolerance to any of a variety of environmental stresses. Many crop plants are transgenic comprising recombinant DNA that confers herbicide and/or pest resistance traits. Incorporation of additional recombinant DNA for conferring crop improvement traits in crop plants presents a challenge of using DNA constructs of increased complexity.

### Summary of the Invention

This invention provides a hybrid corn seed with stacked engineered traits. Such hybrid corn seed having in its genome:
(a) heterologous recombinant DNA which confers a crop improvement trait and herbicide resistance, wherein said crop improvement trait is water deficit tolerance conferred by a recombinant heterologous DNA construct comprising a gene which encodes a Hap3 protein having an amino acid sequence identical to the consensus amino acid sequence of SEQ ID NO:8 or SEQ ID NO:9, and
(b) heterologous recombinant DNA which confers an herbicide resistance trait and a pest resistance trait.

The invention further provides a plant having recombinant DNA that confers both a crop improvement trait and a herbicide and/or pest resistance trait, wherein said crop improvement trait is water deficit tolerance conferred by a recombinant heterologous DNA construct comprising a gene which encodes a Hap3 protein having an amino acid sequence identical to the consensus amino acid sequence of SEQ ID NO:8 and/or having at least one of the terminus amino acid consensus sequences of SEQ ID NO:9 or 10.

### Brief Description Of The Drawings

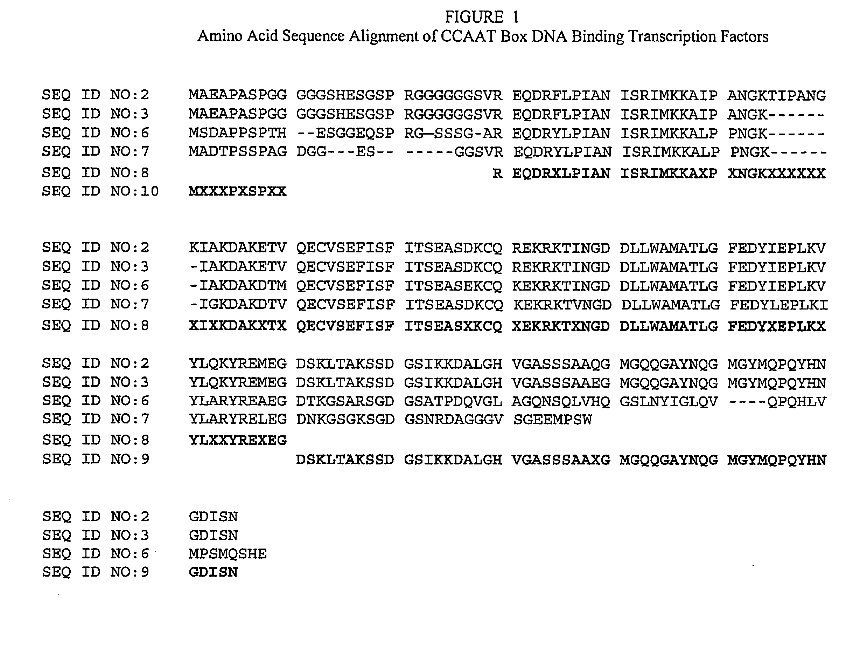

Figure 1 is an amino acid sequence alignment.

### Detailed Description Of The Invention

SEQ ID NO: 1 provides the DNA sequence for an exogenous transcriptional unit comprising promoter elements, DNA encoding a *Zea mays* transcription factor and terminator elements.

SEQ ID NO:2 provides the amino acid sequence of a transcription factor encoded by the DNA encoding a *Zea* mays transcription factor within the exogenous transcriptional unit of SEQ ID NO:1. The transcription factor is described as a CCAAT-box DNA binding protein subunit B.

SEQ ID NO:3 provides the amino acid sequence for a *Zea mays* transcription factor that is homologous to the transcription factor with the amino acid sequence of SEQ ID NO:2. The transcription factor of SEQ ID NO:3 was originally disclosed by Li et al. in Nucleic Acid Res. 20(5), 1087-1091 (1992).

SEQ ID NO:4 provides the DNA sequence of a *Zea mays* gene that encodes the transcription factor of SEQ ID NO:3.

SEQ ID NO:5 provides DNA sequence of a *Glycine max* gene that encodes a transcription factor that is homologous to the *Zea mays* transcription factors of SEQ ID NO:2 and 3.

SEQ ID NO:6 provides the amino acid sequence for the a *Glycine max* transcription factor that is encoded by the DNA of SEQ ID NO:5.

SEQ ID NO:7 provides the amino acid sequence of an *Arabidopsis thaliana* transcription factor that is homologous to the *Zea mays* transcription factors of SEQ ID NO:2 and 3.

SEQ ID NO:8 is an artificial consensus amino acid sequence of a common core region of amino acids of the transcription factors of SEQ ID NO:2, 3, 6 and 7.

SEQ ID NO:9 is an artificial consensus amino acid sequence of a common terminus region of the *Zea mays* transcription factors of SEQ ID NO:2 and 3.

SEQ ID NO:10 is an artificial consensus amino acid sequence of a common terminus region of eight amino acids of the transcription factors of SEQ ID NO:2, 3, 6 and 7.

As used herein "crop improvement trait" means a trait that produces a crop yield or quality improvement, e.g. yield, *per se,* such as increased seed number and/or increased seed weight; tolerance to an environmental stress such as cold, heat, plant density or water deficiency; tolerance to a nutrient deficiency such a nitrogen or phosphorus deficiency; a plant growth and development improvement such as decreased abscission, delayed vegetative senescence, early maturity, increased plant size, increased plant growth rate, increased biomass, enhanced photosynthesis under low light and increased fertility; improved seed quality such as decreased ovule abortion, enhanced endosperm cell number or size, enhanced number or size of leaf or seed starch granules and increased oil, protein or starch accumulation; improved source efficiency such as improved photosynthesis efficiency, increased sugar accumulation, increases starch accumulation, increased sugar export, increases carbon and nitrogen partitioning and increased amino acid transport; improved crop quality such as an increase in one or more amino acids, an increase in seed carbohydrate, protein or oil levels and an increase in total seed oil and protein levels.

As used herein "water deficit" is a plant condition characterized by water potential in a plant tissue of less than -0.7 megapascals (MPa), e.g. -0.8 Mpa. Water potential in maize is conveniently measured by clamping a leaf segment in a pressurizable container so that a cut cross section of leaf is open to atmospheric pressure. Gauge pressure (above atmospheric pressure) on the contained leaf section is increased until water begins to exude from the atmospheric-pressure-exposed cross section; the gauge pressure at incipient water exudation is reported as negative water potential in the plant tissue, e.g. 7 bars of gauge pressure is reported as -0.7 MPa water potential. Water deficit can be induced by withholding water from plants for sufficient time that wild type plants are deleteriously affected, e.g. as manifested by reduced yield, stunted growth, retarded development or death. The plants of this invention show a remarkable risibility after periods of water deficit that are adverse to wild type plants.

As used herein "yield" of a crop plant means the production of a crop, e.g. shelled corn kernels or soybean or cotton fiber, per unit of production area, e.g. in bushels per acre or metric tons per hectare, often reported on a moisture adjusted basis, e.g. corn is typically reported at 15.5 % moisture. Moreover a bushel of corn is defined by law in the State of Iowa as 25.4 kg (56 pounds) by weight, a useful conversion factor for corn yield is: 100 bushels per acre is equivalent to 6.272 metric tons per hectare. Other measurements for yield are in common practice.

As used herein a "transgenic" organism, e.g. plant or seed, is one whose genome has been altered by the incorporation of a trait-conferring, recombinant DNA, e.g. exogenous DNA or additional copies of native DNA, e.g. by transformation or by breeding with a transformed plant. Thus, transgenic plants include progeny plants of an original plant derived from a transformation process including progeny of breeding transgenic plants with wild type plants or other transgenic plants. The enhancement of a desired trait can be measured by comparing the trait property in a transgenic plant which has recombinant DNA conferring the trait to the trait level in a progenitor plant. As used herein "progenitor plant" refers to a plant of essentially the same genotype as a transgenic plant but lacking the specific trait-conferring, recombinant DNA that characterizes the transgenic plant. Such a progenitor plant that lacks that recombinant DNA can be a natural, wild-type plant, an elite, non-transgenic plant, or a transgenic plant without the specific trait-conferring, recombinant DNA that characterizes the transgenic plant. The progenitor plant lacking the specific, trait-conferring recombinant DNA can be a sibling of a transgenic plant having the specific, trait-conferring recombinant DNA. Such a progenitor sibling plant may comprise other recombinant DNA.

Crop plants of interest in the present invention include soybean (including the variety known as *Glycine max),* cotton, canola (also known as rape), corn (also known as maize and *Zea mays*), wheat, sunflower, sorghum, alfalfa, barley, millet, rice, tobacco, fruit and vegetable crops and turfgrasses.

As used herein an "herbicide resistance" trait is a characteristic of a transgenic plant that is resistant to dosages of an herbicide that is typically lethal to a progenitor plant Such herbicide resistance can arise from a natural mutation or more typically from incorporation of recombinant DNA that confers herbicide resistance. Herbicides for which resistance is useful in a plant include glyphosate herbicides, phosphinothricin herbicides, oxynil herbicides, imidazolinone herbicides, dinitroaniline herbicides, pyridine herbicides, sulfonylurea herbicides, bialaphos herbicides, sulfonamide herbicides and gluphosinate herbicides. To illustrate the that production of transgenic plants with herbicide resistance is a capability of those of ordinary skill in the art reference is made to U.S. 2003/0106096 and 2002/0112260 and U.S. Patents 5,034,322; 6,107,549 and 6,376,754.

As used herein an "pest resistance" trait is a characteristic of a transgenic plant is resistant to attack from a plant pest such as a virus, a nematode, a larval insect or an adult insect that typically is capable of inflicting crop yield loss in a progenitor plant. Such pest resistance can arise from a natural mutation or more typically from incorporation of recombinant DNA that confers pest resistance. For insect resistance, such recombinant DNA can, for example, encode an insect lethal protein such as a delta endotoxin of *Bacillus thuringiensis* bacteria or be transcribed to a dsRNA targeted for suppression of an essential gene in the insect. To illustrate that the production of transgenic plants with pest resistance is a capability of those of ordinary skill in the art reference is made to U.S. Patents 5,250,515 and 5,880,275 which disclose plants expressing an endotoxin of *Bacillus thuringiensis* bacteria, to U.S. Patent 6,506,599 which discloses control of invertebrates which feed on transgenic plants which express dsRNA for suppressing a target gene in the invertebrate, to U.S. Patent 5,986,175 which discloses the control of viral pests by transgenic plants which express viral replicase, and to U.S. 2003/0150017 which discloses control of pests by a transgenic plant which express a dsRNA targeted to suppressing a gene in the pest.

**Protein and Polypeptide Molecules -** As used herein "protein" means a polypeptide of combined amino acids including a natural protein or polypeptide fragment of a natural protein or a modified natural protein or a synthetic protein, or a peptide having a protein function. Proteins produced and used by the transgenic plants of this invention are whole proteins or at least a sufficient portion of an entire protein to impart the relevant biological activity of the protein, e.g. a crop improvement trait. To illustrate, reference is made to Hap3 proteins which are effective in conferring water deficit tolerance in transgenic plants, e.g. when constitutively expressed. The Hap3 proteins are transcription factors having a CCAAT-box DNA binding domain and include the *Zea mays* transcription factors of SEQ ID NO: 2 and 3, the *Glycine max* transcription factor of SEQ IS NO:6 and the *Arabidopsis thaliana* transcription factor of SEQ ID NO:7 or a functionally-equivalent homologous transcription factor. Such functionally-equivalent homologous transcription factor can be defined by the consensus amino acid sequences that characterize these transcription factors. With reference to Figure 1 the defining consensus sequences are the central amino acid region consensus sequence of SEQ ID NO:8 and at least one of the terminus amino acid consensus sequences of SEQ ID NO:9 and SEQ ID NO:10. Aside from similarity in function homologs of proteins or DNA can be described as molecules having a sequence, e.g. amino acid or nucleotide sequence, which shares identity to a reference sequence. For simplicity, DNA homologs are defined by optimally aligning the nucleotide sequence of a putative DNA homolog with a defined nucleotide sequence and determining identical nucleotide elements over a window of the defined sequence. Similarly, protein homologs of a consensus amino acid sequence is defined by optimally aligning the amino acid sequence of a putative protein homolog with a defined amino acid sequence and determining the identical amino acid elements over a window of the defined sequence. Optimal alignment can be effected manually but more preferably with the assistance of a homology-based search algorithms such as those commonly known and referred to as BLAST, FASTA, and Smith-Waterman.

For other than consensus amino acid sequences protein homologs are defined by optimally aligning the amino acid sequence of a putative protein homolog with a defined amino acid sequence and determining the conservatively substituted amino acid elements over a window of the defined sequence. Conservatively substituting amino acids are (1) acidic (negatively charged) amino acids such as aspartic acid and glutamic acid; (2) basic (positively charged) amino acids such as arginine, histidine, and lysine; (3) neutral polar amino acids such as glycine, serine, threonine, cysteine, tyrosine, asparagine, and glutamine; (4) neutral nonpolar (hydrophobic) amino acids such as alanine, leucine, isoleucine, valine, proline, phenylalanine, tryptophan, and methionine; (5) amino acids having aliphatic side chains such as glycine, alanine, valine, leucine, and isoleucine; (6) amino acids having aliphatic-hydroxyl side chains such as serine and threonine; (7) amino acids having amide-containing side chains such as asparagine and glutamine; (8) amino acids having aromatic side chains such as phenylalanine, tyrosine, and tryptophan; (9) amino acids having basic side chains such as lysine, arginine, and histidine; (10) amino acids having sulfur-containing side chains such as cysteine and methionine. To account for insertions and deletions, mutations, variations in the C and/or N terminal regions of proteins and non-conservative substitutions, protein homologs are defined as being at least 80% identical.

**Recombinant DNA Constructs -** The present invention contemplates the use of polynucleotides which encode a protein effective for imparting resistance and/or tolerance to water deficit in plants. Such polynucleotides are assembled in recombinant DNA constructs using methods known to those of ordinary skill in the art. A useful technology for building DNA constructs and vectors for transformation is the GATEWAY™ cloning technology (available from Invitrogen Life Technologies, Carlsbad, California) uses the site specific recombinase LR cloning reaction of the Integrase/*att* system from bacterophage lambda vector construction, instead of restriction endonucleases and ligases. The LR cloning reaction is disclosed in U.S. Patents 5,888,732 and 6,277,608, U.S. 2001/283529, 2001/282319 and 2002/0007051. The GATEWAY™ Cloning Technology Instruction Manual which is also supplied by Invitrogen also provides concise directions for routine cloning of any desired RNA into a vector comprising operable plant expression elements.

Recombinant DNA constructs used for transforming plant will comprise DNA cells for conferring a trait along with other commonly used DNA elements As is well known in the art such constructs typically also comprise a promoter and other regulatory elements, 3' untranslated regions (such as polyadenylation sites), transit or signal peptides and marker genes elements as desired. For instance, see U.S. Patents No. 5,858,742 and 5,322,938 which disclose versions of the constitutive promoter derived from cauliflower mosaic virus (CaMV35S), U.S. Patent 6,437,217 which discloses a maize RS81 promoter, U.S. Patent 5,641,876 which discloses a rice actin promoter, U.S. Patent 6,426,446 which discloses a maize RS324 promoter, U.S. Patent 6,429,362 which discloses a maize PR-1 promoter, U.S. Patent 6,232,526 which discloses a maize A3 promoter, U.S. Patent 6,177,611 which discloses constitutive maize promoters, U.S. Patent 6,433,252 which discloses a maize L3 oleosin promoter, U.S. Patent 6,429,357 which discloses a rice actin 2 promoter and intron, U.S. Patent 5,837,848 which discloses a root specific promoter, U.S. Patent 6,084,089 which discloses cold inducible promoters, U.S. Patent 6,294,714 which discloses light inducible promoters, U.S. Patent 6,140,078 which discloses salt inducible promoters, U.S. Patent 6,252,138 which discloses pathogen inducible promoters, U.S. Patent 6,175,060 which discloses phosphorus deficiency inducible promoters, U.S. 2002/0192813 which discloses 5', 3' and intron elements useful in the design of effective plant expression vectors, WO99/58659 which discloses a coixin promoter, WO02/57471 which discloses a maize chloroplast aldolase promoter.

In many aspects of the invention it is preferred that the promoter element in the DNA construct should be capable of causing sufficient expression to result in the production of an effective amount of the transcription factor in water deficit conditions. Such promoters can be identified and isolated from the regulatory region of plant genes which are over expressed in water deficit conditions. Alternatively, such promoters can be exogenous constitutive promoters. Another class of useful promoters are water-deficit-inducible Specific water-deficit-inducible promoters for use in this invention are derived from the 5' regulatory region of genes identified as a heat shock protein 17.5 gene (*HSP17.5*), an HVA22 gene (*HVA22*), and a cinnamic acid 4-hydroxylase (CA4H) gene (*CA4H*) of *Zea maize*; such water-deficit-inducible promoters are disclosed in U.S. 2004/123347 (claiming priority of U.S. provisional application 60/435,987, filed December 20, 2002). Another water-deficit-inducible promoter is derived from the *rab-17* promoter as disclosed by Vilardell et al., Plant Molecular Biology, 17(5):985-993, 1990.

In general it is preferred to introduce heterologous DNA randomly, i.e. at a non-specific location, in the plant genome. In special cases it may be useful to target heterologous DNA insertion in order to achieve site specific integration, e.g. to replace an existing gene in the genome. In some other cases it may be useful to target a heterologous DNA integration into the genome at a predetermined site from which it is known that gene expression occurs. Several site specific recombination systems exist which are known to function implants include cre-lox as disclosed in U.S. Patent 4,959,317 and FLP-FRT as disclosed in U.S. Patent 5,527,695.

Constructs and vectors may also include a transit peptide for targeting of a gene target to a plant organelle, particularly to a chloroplast, leucoplast or other plastid organelle. For a description of the use of a chloroplast transit peptide see U.S. Patent 5, 188,642.

In practice DNA is introduced into only a small percentage of target cells in any one transformation experiment. Marker genes are used to provide an efficient system for identification of those cells that are stably transformed by receiving and integrating a transgenic DNA construct into their genomes. Preferred marker genes provide selective markers which confer resistance to a selective agent, such as an antibiotic or herbicide. Any of the herbicides to which plants of this invention may be resistant are useful agents for selective markers. Potentially transformed cells are exposed to the selective agent. In the population of surviving cells will be those cells where, generally, the resistance-conferring gene is integrated and expressed at sufficient levels to permit cell survival. Cells may be tested further to confirm stable integration of the exogenous DNA. Commonly used selective marker genes include those conferring resistance to antibiotics such as kanamycin (*nptII*), hygromycin B (*aph IV*) and gentamycin (*aac3* and *aac*C4) or resistance to herbicides such as glufosinate (*bar* or *pat*) and glyphosate (EPSPS). Examples of such selectable are illustrated in U.S. Patents 5,550,318; 5,633,435; 5,780,708 and 6,118,047. Screenable markers which provide an ability to visually identify transformants can also be employed, *e*.*g*., a gene expressing a colored or fluorescent protein such as a luciferase or green fluorescent protein (GFP) or a gene expressing a *beta*-glucuronidase or *uid*A gene (GUS) for which various chromogenic substrates are known. **Crop improvement traits** - This invention employs recombinant DNA constructs in providing the transgenic seeds for growing a transgenic plant with a crop improvement trait. Such a transgenic plant has a crop improvement trait stacked with an herbicide resistance trait and/or a pest resistance trait. Such a transgenic plant has improved crop trait as compared to a progenitor plant not having recombinant DNA which codes for the crop improvement trait. Crop improvement traits include cold tolerance, improved nitrogen processing, improved phosphorus use efficiency, reduced abscission, early maturity, enhanced growth and leaf development, enhanced growth rate, low light tolerance, optimized C3 to C4 transition, improved plant development, increased plant size, population stress tolerance, reduced plant size, improved seed germination, delayed senescence, increased amino acid content, increased seed protein, increased seed oil, increased seed starch biosynthesis, increased endosperm cell number and/or size, increased vitamin content, increased photosynthesis efficiency, increased source enhancement, enhanced transport of amino acids, enhanced water deficit tolerance. Such crop improvement traits can provide yield improvement. For instance, when exposed to adverse water deficit conditions, plants with improved water deficit tolerance exhibit improved yield as compared to progenitor plants not having recombinant DNA which confers the water deficit tolerance trait. A useful crop improvement trait for stacking with herbicide and/or pest resistance traits is cold tolerance, e.g. as indicated by increased plant recovery rate or plant survival rate after growth under freezing temperatures or increased germination and seedling vigor at temperature below 10 °C and/or cold and wet conditions. See table 1 for genes that can be useful for achieving cold tolerance, e.g. when over expressed in a recombinant DNA construct.

**Table 1**

| Cold tolerance genes | Reference |
|---|---|
| CBF transcription factors | Nature Biotechnology (1999) 17,287-291. |
| CBF1 | Science. 1998 Apr 3;280(5360):104-6. |
| ICE1 | Genes Dev. 2003 Apr 15;17(8):1043-54. Epub 2003 Apr 02. |
| CodA | Plant J 1998 Oct;16(2):155-61; Plant J 1997 Jul;12(1):133-42; Plant J 2000 Jun;22(5):449-53. |
| GS2 | Plant Mol Biol. 2000 May;43(1):103-11. |
| COR15a | Proc Natl Acad Sci USA. 1996 Nov 12;93(23):13404-13409; Proc Natl Acad Sci USA. 1998 Nov 24;95(24):14570-5. |
| FAD7; FAD8 | Kodama H et al. (1994) Plant Physiol. 105(2):601-605. Kodama et al. (1995) Plant Physiol. 107: 1177-85. Murakami et al. (2000) Science 287: 476-479). Bertin et al. (1998) Plant Growth Reg. 24: 31-41. |
| ABI3 | Plant J. 2001 Jan;25(1):1-8. |
| CBF3 | Plant Physiol. 2000 Dec;124(4):1854-65. Plant Cell. 2001 Jan;13(1):61-72. |
| OsCDPK7 | Plant Cell Physiol. 2001 Nov;42(11):1228-33. |
| NtSCOF-1 | Mol Cells. 2001 Oct 31;12(2):204-8 ; Plant J. 2001 Feb;25(3):247-59. |
| AtPP2CA | Plant J. 2001 May;26(4):461-70; J Exp Bot. 2001 Jan;52(354):181-2. |
| AtGST/GPX | Nat Biotechnol. 1997 Oct;15(10):988-91. |
| NtSOD | Plant Physiol. 1993 Dec;103(4):1067-1073. |
| pBE2113/hiC6 | Biosci Biotechnol Biochem 2001 Aug;65(8):1796-804. |
| OsWX | Mol Biol Evol. 1998 Aug;15(8):978-87. |
| ADH | Plant Physiol. 111: 381-391; Plant Physiol. 105: 1075-1087. |
| AtHK1 | FEBS Lett. 427: 175-178. |
| ADS2 | Plant Cell Physiol. 39: 247-253. |
| RD29A | Plant Physiol. 103: 1047-1053; Plant Mol. Biol. 21: 641-653 ; Mol. Gen. Genet. 236: 331-340. |
| XERO1 | FEBS Lett. 381: 252-256. |
| P5CS1 | Plant J. 12: 557-569. |
| P5CS2 | Plant J. 12: 557-569. |
| PAL | Plant Physiol. 108: 39-46. |
| PDC1 | Plant Physiol. 119: 599-607. |
| PUMP | FEBS Letts. 429: 403-406. |
| RCI3 | Plant J. 2002 Oct;32(1):13-24. |
| NDPK2 | Proc Natl Acad Sci U S A 2003 Jan 7;100(1):358-63. |
| GPAT | Plant Cell Physiol. 2002 Jul;43(7):751-8; Physiol Plant 2003 May; 118(1):57-63. |
| AtCCT2 | Plant Cell Physiology 0043 01342-01350; Mol Cells. 28;11(1):95-9; Mol Cells. 1997 Feb 28;7(1):58-63. |
| AZF1, AZF2, AZF3, STZ, GCN5 | Gene 248:23-32, 2000. |
| Lipid desaturase | Nature Biotechnology (1996) 14, 1003-1006. |
| *AtSAC1* | Plant J 34, 293-306 (2003). |

A useful crop improvement trait for stacking with herbicide and/or pest resistance traits is nitrogen processing, e.g. the ability of a plant to fix or reduce nitrogen from N₂ into NO₃, NH₃ or organic molecules. Nitrogen processing can also be characterized as low nitrogen tolerance as indicated by increased plant growth rate, biomass, seed set, photosynthesis under growth limiting nitrogen conditions. Nitrogen processing can also be characterized as enhanced nitrogen uptake, translocation, storage or seed partitioning. See table 2 for genes that can be useful for nitrogen processing, e.g. when over expressed in a recombinant DNA construct.

**Table 2**

| Nitrogen processing | Reference |
|---|---|
| Nodulin-35 (uricase) | Plant J. 3: 599-606, 1993. |
| G225 | U.S. 2004/006797 |
| NDP kinase | PNAS (2003) 100: 358-363. |
| iscN | Molecular Genetics Genomics (2002) 267: 820-828. |
| HIUHase. | Plant Physiology 0130 02061-02068 2002. |
| Glutamine synthetase 2 | Migge et al. (2000). Planta 210, 252-260. |
| Nitrogenase in chloroplasts | Plant and Soil (1997) 193-203. |
| Asparagine synthetase | Lam et al. (2003). Plant Physiol. 132, 926-935. |
| AtUPS1 | The Plant Cell (2002) 14: 847-856. |
| AtDUR3 | The Plant Cell (2003) 15: 790-800. |
| AtNRT1;1 | The Plant Cell (2003) 15: 107-117. |
| OsAMT1;1 to OsAMT1;3 | Plant Cell Physiol. (2003) 44(7):726-34. |
| asparagine synthase | several patents in this area |
| Glutamine synthetase 1 | Oliveira et al. (2002). Plant Physiol. 129, 1170-1180. |

A useful crop improvement trait for stacking with herbicide and/or pest resistance traits is improved phosphorus use efficiency, e.g. enhanced phosphorus uptake, translocation or vacuolar concentration. The tomato phosphate transporter gene (*LePT3*) as disclosed in WO01/55299 can be useful for achieving improved phosphorus use efficiency in plants, e.g. when over expressed in a recombinant DNA construct.

A useful crop improvement trait for stacking with herbicide and/or pest resistance traits is reduced abscission, e.g. the absence of flower petal abscission, seed dehiscence/shatter or silique splitting. See table 3 for genes that can be useful for achieving reduced abscission in plants, e.g. when over expressed in a recombinant DNA construct.

**Table 3**

| Reduced abscission | Reference |
|---|---|
| *ALC* | Curr. Biology 11, 1914-1922 (2001). |
| *HAESA* | Genes Dev.14(1):108-17 (2000). |
| SHP1 | Nature 404; 766-770 ; 2000. |
| JOINTLESS | Nature 406 910-913: 2000. |

A useful crop improvement trait for stacking with herbicide and/or pest resistance traits is early maturity, e.g. early or accelerated seed dry down or silique senescence with normal onset of flowering. *GPA1 and OST1* genes with seed/husk specific expression as disclosed in J. Exp.Bot., 51,447-458 and Science, 292:2070-2 and Trends Plant Sci., 8:151-3 can be useful for achieving early maturity in plants, e.g. when over expressed in a recombinant DNA construct.

A useful crop improvement trait for stacking with herbicide and/or pest resistance traits is enhanced growth and leaf development. The *Oshox1* gene as disclosed in Plant J., 11(2):263-76 can be useful for achieving enhanced growth and leaf development in plants, e.g. when over expressed in a recombinant DNA construct.

A useful crop improvement trait for stacking with herbicide and/or pest resistance traits is enhanced growth rate without causing significant premature flowering. The *PAUSED* gene as disclosed in Plant Physiol, 132(4):2135-43 (Aug. 2003) can be useful for achieving such enhanced growth rate in plants, e.g. when over expressed in a recombinant DNA construct.

A useful crop improvement trait for stacking with herbicide and/or pest resistance traits is low light tolerance, e.g. as characterized by plants which exhibit insensitivity to low light stress or altered spectral quality stress or enhanced photosynthesis under low light conditions. See table 4 for genes that can be useful for achieving low light tolerance in plants, e.g. when over expressed in a recombinant DNA construct.

**Table 4**

| Low light tolerance | Reference |
|---|---|
| ARR4. | Plant Physiology, 128: 363-369 (2002). |
| CRY2 | Cell,103:815-827. |
| PHYB | Wagner et al., Plant Cell 3, 1275-1288; Short, Plant Physiol. 119, 1497-1505; Halliday et al., Plant J. 12, 1079-1090; Thiele et al., Plant Physiol. 120, 73-81; Jackson et al., Plant J. 9, 159-166. |

A useful crop improvement trait for stacking with herbicide and/or pest resistance traits is optimized C3 to C4 transition, e.g. as characterized by plants having an operational C4 pathway in C3 plants or the establishment of Kranz anatomy, which results in increased plant vigor or growth rate. See table 5 for genes that can be useful for achieving optimized C3 to C4 transition in plants, e.g. when over expressed in a recombinant DNA construct.

**Table 5**

| Optimized C3 to C4 transition | Reference |
|---|---|
| C4PPDK | PNAS (1993) 90: 9586. |
| PEPC | Nat. Biotechn. (1999) 17:76. |
| C4 Phospho*enol*pyruvate carboxylase | Plant Production Science (2001) 4: 311 - 312. |

A useful crop improvement trait for stacking with herbicide and/or pest resistance traits is improved plant development, e.g. as characterized by control of meristem patterning to increase the number of fertile flowers, number of fertilized ovules, organ number or yield. The *Blind gene* as disclosed in PNAS, 99, 1064-1069 (2002) can be useful for achieving such improved plant development, e.g. when over expressed in a recombinant DNA construct.

A useful crop improvement trait for stacking with herbicide and/or pest resistance traits is improved plant growth, e.g. as indicated by cell expansion. The *UCU1* gene as disclosed in Dev.Biol., 242, 161-173 (2202) can be useful for achieving such improved plant growth, e.g. when over expressed in a recombinant DNA construct.

A useful crop improvement trait for stacking with herbicide and/or pest resistance traits is improved plant productivity, e.g. as characterized by increased root and shoot growth rates, increased biomass and harvest index, increased seed size and number, increased seed yield and/or increased seed oil content. See table 6 for genes that can be useful for achieving improved plant productivity, e.g. when over expressed in a recombinant DNA construct.

**Table 6**

| Improved plant productivity | Reference |
|---|---|
| AGL8 | U.S. Patent 6,229,068. |
| ANT | U.S. 2002/0170093. |

A useful crop improvement trait for stacking with herbicide and/or pest resistance traits is increased plant size, e.g. as characterized by increased cell size or cell number in organs such as seed, leaf, flower, roots, vascular bundles. See table 7 for genes that can be useful for achieving improved plant productivity, e.g. when over expressed in a recombinant DNA construct.

**Table 7**

| Increased plant size | Reference |
|---|---|
| ANT | U.S. 2002/0170093. |
| ATHB6 (Transcription factor) | Plant Mol Biol. 1999 Aug;40(6):1073-83. |
| 4CL | Lee, D., et al., (1997) Plant Cell 9: 1985-1999. |
| ARGOS | Plant cell 15, 1951-1961 (2003). |
| *TFL1* | Dev.125, 1609-1615.(1998). |
| DWF4 | Choe et al. (2001) Plant Journal 26: 573-582. |
| GA20 oxidase | Carrera et al., Plant J. 22, 247-256; Coles et al., Plant J. 17, 547-556. |
| plant hemoglobin genes | Plant Journal (2003) 35: 763-770; PNAS (1998) 95: 10317-10321; Nature Biotech 15: 244-247. |

A useful crop improvement trait for stacking with herbicide and/or pest resistance traits is population stress tolerance, e.g. as characterized by increased and/or uniform biomass, root mass, growth rate, photosynthesis, seed set or yield under population stress. The *AtHB2gene* as disclosed in Development, 126:4235-4245 (1999) and the *PHYB* gene as disclosed in Nature Biotechnology 17,287-291 (1999) can be useful for achieving population stress tolerance in plants, e.g. when over expressed in a recombinant DNA construct.

A useful crop improvement trait for stacking with herbicide and/or pest resistance traits is improved seed germination. The *DAG1* gene as disclosed in Plant physiology 128(2):411-7 and the *AtCYP51* gene as disclosed in Biochem Biophys Res Commun 6: 285(1):98-104 can be useful for achieving improved seed germination, e.g. when over expressed in a recombinant DNA construct.

A useful crop improvement trait for stacking with herbicide and/or pest resistance traits is short plants, e.g. as characterized by reduced internode length. The *CRY1* gene as disclosed in Plant Cell 13(12):2573-87, the *AtGA2ox8* gene as disclosed in Plant Cell 15(1):151-63 and the *GA-20 oxidase* gene as disclosed in Plant Physiol 126,97-107 can be useful for achieving short plants, e.g. when over expressed in a recombinant DNA construct.

A useful crop improvement trait for stacking with herbicide and/or pest resistance traits is delayed senescence, e.g. as characterized by plants with delayed vegetative senescence together with extended photosynthesis and/or without a delay in the onset of flowering. See table 8 for genes that can be useful for achieving delayed senescence in plants, e.g. when over expressed in a recombinant DNA construct.

**Table 8**

| Delayed senescence | Reference |
|---|---|
| DWF4 expression in leaves | Plant Journal 26: 573-582. |
| KNOTTED 1 | Plant Cell. 1999 Jun;11(6):1073-80. |
| ARR2 | Nature, 413: 383-389.(2001). |
| G571 | WO03/014327. |
| ORE9 | Plant Cell 13(8):1779-90; 2001. |
| PRPS17/ORE4 | Plant J. 31, 331-340; 2002. |
| AGL15 | Plant Cell. 12:183-98; 2000. |
| PAO | Journal of Experimental Botany :53;801-808: 2002. |
| SAG13 | WO03/96797. |
| *G1050* | WO03/014327. |
| SAG promoter w/IPT gene | Science 270:1986-8. |

A useful crop improvement trait for stacking with herbicide and/or pest resistance traits is increased amino acid content in seed, e.g. without a decrease in protein or oil. The *CGS1* gene as disclosed in Plant physiology 128:97-107 can be useful for achieving improved seed germination, e.g. when over expressed in a recombinant DNA construct.

A useful crop improvement trait for stacking with herbicide and/or pest resistance traits is increased seed protein, e.g. without an reduction in oil components. The asparagine synthase gene can be useful for achieving increased seed protein, e.g. when over expressed in a recombinant DNA construct(see also US provisional application Serial No. 60/479,962, filed June 19,2003 for other genes which are useful for increased seed protein when over expressed in a recombinant DNA construct).

A useful crop improvement trait for stacking with herbicide and/or pest resistance traits is increased seed oil, e.g. without a reduction in protein components. The *AtNHX1* gene as disclosed in Proc.Nat..Acad.Sci.USA, 98,12832-12836 and genes disclosed in WO05/24017 (claiming priority of U.S. 10/613,520, filed July 2, 2003) including pyruvate kinase and phosphofructokinase can be useful for achieving increased seed oil, e.g. when over expressed in a recombinant DNA construct.

A useful crop improvement trait for stacking with herbicide and/or pest resistance traits is increased vitamin content in plant, e.g. beta carotene. The *crtB* gene as disclosed in Proc.Nat.Acad.Sci.USA, 99,1092-1097 can be useful for achieving increased vitamin content, e.g. when over expressed in a recombinant DNA construct.

A useful crop improvement trait for stacking with herbicide and/or pest resistance traits is increased starch biosynthesis, e.g. increases sucrose hydrolysis in kernel endosperm that results in increase in starch biosynthesis and endosperm and kernel size. The sucrose phosphorylase, *gftA sp1* gene as disclosed in US patents 6,235,971 and 6,476,295 can be useful for achieving increased starch biosynthesis, e.g. when over expressed in a recombinant DNA construct.

A useful crop improvement trait for stacking with herbicide and/or pest resistance traits is increased endosperm cell number and/or size, e.g. without adverse effects on seed set or germination. See table 9 for genes that can be useful for achieving delayed senescence in plants, e.g. when over expressed in a recombinant DNA construct.

**Table 9**

| Increased endosperm cell number and/or size | Reference |
|---|---|
| MEDEA | Plant Cell 12, 2367-2381. |
| FIE | PNAS 97(19):10637-42. (2002). |
| DME | Cell 2002: 110, 33-42. (2002). |
| MSI1 | EMBO J. 22(18):4804-4814. (2003). |
| ANT | U.S. 2002/0170093. |
| ETR1-1 | WO 95/01439; Plant Physiol. 115: 737-751; Plant Mol Biol 44: 283-301. |
| APL1, APL2, APL3 and APL4 | Journal of Biological Chemistry 0278: 28508-28515 2003. |
| fus3/lec mutants | Development 128(2):243-52. (2001) |

A useful crop improvement trait for stacking with herbicide and/or pest resistance traits is increased photosynthetic efficiency, e.g. increased total carbon fixation rate in plants under either normal or saturated light and carbon dioxide environments. The *CCA1* gene as disclosed in Cell 93(7):1207-17 can be useful for achieving increased photosynthetic efficiency, e.g. when over expressed in a recombinant DNA construct.

A useful crop improvement trait for stacking with herbicide and/or pest resistance traits is increased source enhancement, e.g. as characterized by increased sugar accumulation, increased starch accumulation and/or sucrose export. See table 10 for genes that can be useful for achieving delayed senescence in plants, e.g. when over expressed in a recombinant DNA construct.

**Table 10**

| Increased source enhancement | Reference |
|---|---|
| StcPGM (cytosolic isoform of phosphoglucomutase) | Planta (2002) 214:510-520. |
| SoSUT1 | Planta. 2003 May;217(1):158-67. |
| CCA1 | US provisional application No. 60/494397 |
| Sucrose Phosphate Synthase | Planta (2001) 212: 817-822 and numerous external and internal reports. |
| FBPase/SBPase bifunctional | Nature Biotech. 19: 965-969; 2001. |
| *VfAGP* | Planta 214(6), 954-64 (2002). |
| ELIP1 (Early light-induced protein1) | PNAS:100; 4921-04926, 2003. |
| PsbS / NPQ4 Nonphotochemical Quenching 4 | PNAS ;99: 15222-15227, 2002. |

A useful crop improvement trait for stacking with herbicide and/or pest resistance traits is enhanced transport of amino acids, e.g. as characterized by increased accumulation or transport of glutamic acid, glutamine, aspartic acid or asparagine. See table 11 for genes that can be useful for achieving enhanced transport of amino acids, e.g. when over expressed in a recombinant DNA construct.

**Table 11**

| enhanced transport of amino acids | Reference |
|---|---|
| AAP1 | PNAS (1993) 90: 5944-5948. |
| AAP2 | Plant Journal (1993) 4:993-1002. |
| AAP3 | J Biol Chem (1994) 27: 16315-16320. |
| AAP4 | J Biol Chem (1994) 27: 16315-16320. |
| AAP5 | J Biol Chem (1994) 27: 16315-16320. |
| AAP6 | Plant Cell (1996) 8: 1437-1446. |
| AAP7 | J Biol Chem. (2002) 277:45338-45346. |
| AAP8 | J Biol Chem (2002) 277:45338-45346. |
| AUX1 | Science (1996) 273: 948-950. |
| ProT | Plant Cell (1996) 8: 1437-1446. |
| LHT1 | Plant Physiology (1997) 115: 1127-1134. |
| ANT1 | Plant Physiology (2001) 125: 1813-1820. |
| amino acid transporter | Arabidopsis TxP experiment for G1543 transgenics. PHEN280. |
| Beta-GDH | Internal data. |

A useful crop improvement trait for stacking with herbicide and/or pest resistance traits is enhanced water deficit tolerance, e.g. as characterized by resistance and/or tolerance to water deficit, dehydration resulting from exposure to saline water, freezing conditions, low humidity and/or recovery after water deprivation. See table 12 for genes that can be useful for achieving enhanced water deficit tolerance, e.g. when over expressed in a recombinant DNA construct.

**Table 12**

| Water deficit tolerance | Reference |
|---|---|
| SOD(Mn superoxide dismutase) | Maria et al., 2002. |
| IMT1 (Myo-inositol o-methyltransferase (D-ononitol synthesis)) | Plant Physiol, 115, (3) 1211-1219 (1997). |
| CBF4 (Transcription factor) | Plant Physiol.; 130(2):639-48 (2002). |
| mt1D (Mannitol-1-phosphate dehydrogenase (mannitol synthesis)) | Plant Physiol.131(4):1748-55 (2003). |
| CRE1 | The Journal of Cell Biology 161 (6);1035-1040. |
| NtAQP1 (PIP1 plasma membrane aquaporin) | Plant Cell. 2002 Apr;14(4):869-76. |
| otsA (Trehalose-6-phosphate synthase (trehalose synthesis) | Plant Physiol. 152, 525-532 (1998). |
| otsB (Trehalose-6-phosphate synthase (trehalose synthesis) | Plant Physiol. 152,525-532 (1998). |
| *NADPH-dependent aldose*/*aldehyde reductase* | Plant J.24(4):437-46 (2000). |

| Water deficit tolerance | Reference |
|---|---|
| PLD alpha (Phospholipase D(alpha) expression) | Plant J. 2001 Oct;28(2):135-44. |
| OsCDPK7(Transcription factor) | Plant J. 2000 Aug;23(3):319-27. |
| Transcription factors | U.S. patent 7,888,557. |
| GB1 | WO04/101741. |
| SOD | Plant Physiol. 1996 Aug;111(4):1177-1181. |
| BADH-1 (Betaine aldehyde dehydrogenase) | Soil Science and Plant Nutrition ,46 (4), 873-884 (2000). |
| Adc (Arginine decarboxylase) | Capell et al. 1998. |
| ME (NADP-malic enzyme which converts malate and NADP to pyruvate, NADPH, and CO2) | Laporte et al. 2002. |
| AtGolS2 (Galactinol and raffinose accumulation) | Plant J. ;29(4):417-26. (2002). |
| AtNCED3 (Increased ABA synthesis) | Plant J. 2001 Aug;27(4):325-33. |
| AtHAL3a (Phosphoprotein phosphatase) | Plant J.20, 529-539 (1999). |
| Apo-Inv (Apoplastic invertase) | Plant and Cell Physiology, 42, (2) 245-249. |
| NPH1/PHOT1 | NATURE, 414: 656:660. |
| GPA1 | Science 292,2070-2072. |

A useful crop improvement trait for stacking with herbicide and/or pest resistance traits is enhanced heat tolerance. See table 13 for genes that can be useful for achieving enhanced heat tolerance, e.g. when over expressed in a recombinant DNA construct.

**Table 13**

| heat tolerance | Reference |
|---|---|
| Apx1 | Gene. 2001 Jul 25;273(1):23-7. |
| Hsp101 | Plant Mol Biol. 2003 Mar;51(4):543-53. |
| *ATHSP101* | PMB 51, 677-686, 2003. |
| *ChyB* | Nature 418(6894):203-6 (2002). |
| hs | Plant J. 1995 Oct;8(4):603-12. |
| TLHS1 | Mol Cells. 31;10(5):519-24. |

**Transformation Methods and Transgenic Plants -** Methods and compositions for transforming plants by introducing a recombinant DNA construct into a plant genome in the practice of this invention can include any of the well-known and demonstrated methods. Preferred methods of plant transformation are microprojectile bombardment as illustrated in U.S. Patents 5,015,580; 5,550,318; 5,538,880; 6,160,208; 6,399,861 and 6,403,865 and *Agrobacterium*-mediated transformation as illustrated in U.S. Patents 5,635,055; 5,824,877; 5,591,616; 5,981,840 and 6,384,301 (see also U.S. patent 6,717,034 for a description of vectors, transformation methods, and production of transformed *Arabidopsis thaliana* plants where transcription factors are constitutively expressed by a CaMV35S promoter).

Transformation methods of this invention to provide plants with enhanced environmental stress tolerance are preferably practiced in tissue culture on media and in a controlled environment. "Media" refers to the numerous nutrient mixtures that are used to grow cells *in vitro*, that is, outside of the intact living organism. Recipient cell targets include meristem cells, callus, immature embryos and gametic cells such as microspores, pollen, sperm and egg cells. It is contemplated that any cell from which a fertile plant may be regenerated is useful as a recipient cell. Callus may be initiated from tissue sources including, but not limited to, immature embryos, seedling apical meristems, microspores and the like. Those cells which are capable of proliferating as callus also are recipient cells for genetic transformation. Practical transformation methods and materials for making transgenic plants of this invention, e.g. various media and recipient target cells, transformation of immature embryos and subsequent regeneration of fertile transgenic plants are disclosed in U.S. Patents 6,194,636 and 6,232,526 and WO02/57471.

The seeds of this invention can be harvested from fertile transgenic plants and be used to grow progeny generations of transformed plants of this invention including hybrid plants line comprising the DNA construct expressing a transcription factor which provides the benefits of resistance and/or tolerance to water deficit.

Having now generally described the invention, the same will be more readily understood through reference to the following examples. These examples illustrates the use of polynucleotides encoding a water-deficit tolerance-imparting transcription factor to provide various transgenic plants exhibiting enhanced tolerance for and/or resistance to growing conditions of water deficit.

### Breeding of Transgenic Plants

In addition to direct transformation of a plant with a recombinant DNA construct, transgenic plants can be prepared by crossing a first plant having a recombinant DNA construct with a second plant lacking the construct. For example, recombinant DNA can be introduced into a plant line that is amenable to transformation to produce a transgenic plant which can be crossed with a second plant line to introgress the recombinant DNA into the second plant line.

In one aspect of the invention a transgenic plant with recombinant DNA conferring a crop improvement trait is crossed with a transgenic plant having recombinant DNA conferring herbicide and/or pest resistance to produce progeny plants having recombinant DNA that confers both the crop improvement trait and the herbicide and/or pest resistance trait. Preferably, in such breeding for combining traits the transgenic plant donating the crop improvement trait is a female line and the transgenic plant donating the herbicide and/or pest resistance trait is a male line. The progeny of this cross will segregate such that some of the plant will carry the DNA for both parental traits and some will carry DNA for one parental trait; such plants can be identified by markers associated with parental recombinant DNA Progeny plants carrying DNA for both parental traits can be crossed back into the female parent line multiple times, e.g. usually 6 to 8 generations, to produce a progeny plant with substantially the same genotype as one original transgenic parental line but for the recombinant DNA of the other transgenic parental line.

In yet another aspect of the invention hybrid transgenic seed, e.g. a hybrid transgenic corn seed, is produced by crossing a female transgenic corn line containing recombinant DNA conferring a crop improvement trait with a male transgenic corn line containing recombinant DNA conferring herbicide and/or pest resistance. In a preferred aspect of this invention hybrid transgenic corn seed is produced by crossing a female transgenic corn line with recombinant DNA conferring both a crop improvement trait and herbicide resistance with a male transgenic corn line with recombinant DNA conferring both herbicide resistance and pest resistance.

### Example 1

This example illustrates aspects of the invention through the production of transgenic corn plants and seed with recombinant DNA that confers water-deficit tolerance.

Transgenic corn was transformed with a recombinant DNA construct having the nucleotide sequence of SEQ ID NO: 1 and which comprises a rice actin 1 constitutive promoter and a rice actin 1 intron operably linked an corn gene which encodes a Hap3 transcription factor with the amino acid sequence of SEQ ID NO:2 followed by a Tr7 3' terminator. The construct further comprised a CaMV 35 S promoter operably linked to an nptII marker gene. Twenty transgenic events in corn were selected as having either 1 or 2 copies of the construct and no oriV origin of replication from the vector. Eleven of the twenty events survived to fertile plants which produced seed. Seed producing plants were analyzed to verify the presence of the exogenous DNA encoding the transcription factor and accumulation of the transcription factor. Six of the transgenic events were used in a water deficit assay.

Pre-germinated seedlings of transgenic plants (progeny of a heterozygous transgenic plant which inherited the exogenous transcription factor DNA construct) and wild type plants (progeny of a heterozygous transgenic plant which did not inherit the exogenous transcription factor DNA construct) were planted in 13 cm (5 inch) pots containing 330 g of soil. The plants were well watered for one week then allowed to dry for 4 d. An equal number (32) of transgenic and wild type plants were selected based on matched height and the selected plants were mixed in eight flats. Four flats were designated as "wet" meaning they would be well watered and four flats were designated as "dry" meaning they would be subject to water deficit. All pots were brought to the weight of the heaviest pot by adding water. The pots were weighed daily until the average pot weight dropped to between 600 to 700 g, whereupon a water deficit assay was started by measuring plant heights and resuming watering for pots in "wet" flats while continuing to withhold water for pots in "dry" flats.

The pots in the "wet" flats were fully-watered daily. The pots in the "dry" flats were weighed daily to determine a water deficit treatment. If the average "dry" flat pot weight was greater than 500 g, no water was added; if the average pot weight was between 365 and 500 g, 35 g of water was added to each pot; and, if the average pot weight was less than 365 g, a determined amount of water was added to bring the average pot weight to 400 g. The water deficit treatment was continued until the pots in the "dry" flats have had an average pot weight below 500 g for 8 d. The height of all plants was measured on the 9^{th} day and averages are reported in Table 14.

**Table 14: Average Change in Plant Height (cm) through first water deficit**

| Treatment | Transgenic | Wild Type | Ratio | Significance |
|---|---|---|---|---|
| Full water | 46.3 cm | 45.5 cm | 1.02 | 0.03 |
| Water deficit | 20.5 cm | 19.7 cm | 1.04 | 0.015 |

On the 9^{th} day full watering was resumed for the "dry" flat pots for 3 d when heights were again measured. See tables 2 and 3 for changes in average plant height for the eight water-deficited plants which were recovered during the 3-day recovery period. Table 15 shows the average incremental change in plant height for the recovered plants which occurred during the 3-day recovery period. Table 16 show the total average change in plant height for the recovered plants which occurred through the combined water-deficit and recovery periods.

**Table 15:Average Change in Recovered Plant Height During 3-day Recovery Period**

| Treatment | Transgenic | Wild Type | Ratio | Significance |
|---|---|---|---|---|
| Water recovered | 13.2 cm | 13.2 cm | 1.0 | 0.74 |

**Table 16: Average Change in Recovered Plant Height Through Deficit and Recovery**

| Treatment | Transgenic | Wild Type | Ratio | Significance |
|---|---|---|---|---|
| Water deficit | 33.8 cm | 32.9 cm | 1.03 | 0.03 |

Recovered plants were subjected to a second round of water deficit as described above. After 9 d full water was resumed for 7 d. After two days of full water the twice water-deficited plants began to show signs of recovery from a wilted state. In some cases recovery took 5-6 d and some plants never recovered. On average the recovered transgenic plants were significantly greener and healthier than recovered wild type plants which were more wilted and yellow (indicating senescence).

### Example 2

This example further illustrates the aspect of this invention relating to transgenic corn. Progeny seed of the transgenic corn produced in Example 1 was planted in a field trial to evaluate its water deficit tolerance as compared to the negative sibling (wild type). The plants were grown in a well irrigated field in Kansas. Water was withheld from half of the planting during the late vegetative stage. The experimental evidence showed that under water deficit conditions transgenic corn plants expressing the Hap3 transcription factor of SEQ ID NO:2 were healthier than the wild type and exhibited the following phenotypes:
(a) likely to have a higher chlorophyll index, e.g. >42 in transgenic plants as compared to <40 in wild type,
(b) likely to produce more photosynthate,
(c) likely to have cooler leaf temperature, and
(d) likely to maintain higher stomatal conductance.

### Example 3

This example illustrates the invention through the preparation of transgenic seed and plants with a crop improvement trait, e.g. water-deficit tolerant soybean.

Transgenic soybean was transformed with a recombinant DNA construct comprising a CaMV 35S constitutive promoter operably linked to an *Arabidopsis thaliana* gene which encodes the Hap3 transcription factor having the amino acid sequence of SEQ ID NO:7 followed by a terminator element. In a water deficit assay Transgenic soybean plants exhibited enhanced resistance to water deficit, i.e. less wilting, as compared to wild type soybean plants. In addition In particular, transgenic plants wilted less, had a higher chlorophyll content, had a higher relative water content, had a higher photosynthesis rate, than their gene negative segregants and parental control plants.

### Example 4

This example illustrates the invention through the preparation of transgenic seed and plants with a crop improvement trait, e.g. water-deficit tolerant soybean.

Transgenic soybean was transformed with a recombinant DNA construct comprising a CaMV 35S constitutive promoter operably linked to an endogenous soybean gene having the nucleotide sequence of SEQ ID NO:5 which encodes the native Hap3 transcription factor having the amino acid sequence of SEQ ID NO:6 followed by a terminator element. In a water deficit assay where water was withheld after saturating potted plants at the V1 stage until the soil reached 10% of capacity (50% for well watered control), transgenic soybean plants exhibited enhanced resistance to water deficit as compared to wild type soybean plants. In particular, transgenic plants wilted less, had a higher chlorophyll content, had a higher relative water content, had a higher photosynthesis rate, than their gene negative segregants and parental control plants.

### Example 5

This example illustrates aspects of the invention through the preparation of transgenic seed and plants with a crop improvement trait and herbicide and insect resistance traits.

Transgenic maize is transformed with recombinant DNA constructs substantially as disclosed in Example 1 except that the selective marker is an EPSPS gene that confers resistance to glyphosate herbicide. The transgenic plants produce seed which can be used to grow water-deficit-tolerant progeny plant which can be bred with transgenic plants having pest resistance to provide progeny plants with stacked engineering traits.

Seed from plants with water deficit tolerance and glyphosate herbicide tolerance are used as female plants in breeding with a pollen from transgenic plants with insect resistance, e.g. maize line MON863 available from Monsanto Company, St. Louis, MO, which was contains recombinant DNA expressing the *cry3Bb1* gene encoding a Coleopteran-specific insecticidal protein from *Bacillus thuringiensis* (subsp. *kumamotoensis*) to control infestation with corn root worm (CRW; *Diabrotica sp*). Segregating progeny plants are selected for all three traits, i.e. water deficit tolerance, herbicide tolerance and insect resistance. Selected plants are back crossed for 6 generations with the water deficit tolerant line. By such breeding the insect resistance trait is introgressed into the transgenic line with water deficit tolerance and glyphosate herbicide tolerance.

### Example 6

This example illustrates another aspect of the invention through the preparation of transgenic seed and plants with a crop improvement trait and herbicide tolerance and insect resistance traits. Transgenic maize is transformed with recombinant DNA constructs substantially as disclosed in Example 1 except that the selective marker is an *bar* gene that confers tolerance to glufosinate herbicide. Seed from water deficit-tolerant, glufosinate herbicide-tolerant plants were used as female plants in breeding with a pollen from a glyphosate herbicide-tolerant, insect-resistant transgenic corn plant, e.g. maize line MON802 available from Monsanto Company, St. Louis, MO and which has recombinant genes encoding the Cry1Ab protein from *Bacillus thuringiensis* and the 5-enolpyruvylshikimate-3-phosphate synthase (EPSPS) from *A*. *tumefaciens* strain CP4. Segregating progeny plants are selected for water deficit tolerance by screening with glufosinate herbicide and insect resistance by screening with glyphosate herbicide.

## Claims

1. A hybrid corn seed having in its genome:
(a) heterologous recombinant DNA which confers a crop improvement trait and herbicide resistance, wherein said crop improvement trait is water deficit tolerance conferred by a recombinant heterologous DNA construct comprising a gene which encodes a Hap3 protein having an amino acid sequence identical to the consensus amino acid sequence of SEQ ID NO:8 or SEQ ID NO:9, and
(b) heterologous recombinant DNA which confers an herbicide resistance trait and a pest resistance trait.

2. The corn seed of claim 1, wherein the hybrid corn seed has resistance to at least one herbicide selected from a glyphosate herbicide, a phosphinothricin herbicide, an oxynil herbicide, an imidazolinone herbicide, a dinitroaniline herbicide, a pyridine herbicide, a sulfonylurea herbicide, a bialaphos herbicide, a sulfonamide herbicide and a gluphosinate herbicide.

3. The corn seed of claim 1, wherein the hybrid corn seed has resistance to at least two herbicides selected from a glyphosate herbicide, a phosphinothricin herbicide, an oxynil herbicide, an imidazolinone herbicide, a dinitroaniline herbicide, a pyridine herbicide, a sulfonylurea herbicide, a bialaphos herbicide, a sulfonamide herbicide and a gluphosinate herbicide.

4. A plant having recombinant DNA that confers both a crop improvement trait and a herbicide and/or pest resistance trait, wherein said crop improvement trait is water deficit tolerance conferred by a recombinant heterologous DNA construct comprising a gene which encodes a Hap3 protein having an amino acid sequence identical to the consensus amino acid sequence of SEQ ID NO:8 and/or having at least one of the terminus amino acid consensus sequences of SEQ ID NO:9 or 10.

5. The plant of claim 4, wherein the plant is a corn, wheat, sunflower, sorghum, alfalfa, barley, millet, rice, tobacco, fruit or vegetable plant.

6. The plant of claim 4 or 5, which has a terminus amino acid sequence identical to a consensus amino acid sequence of SEQ ID NO:10.

## Patentansprüche

1. Hybridmaissamen, der in seinem Genom aufweist:
(a) heterologe rekombinante DNA, die ein Kulturpflanzen-Verbesserungsmerkmal sowie Herbizidresistenz verleiht, wobei es sich bei dem Kulturpflanzen-Verbesserungsmerkmal um Wassermangeltoleranz handelt, die von einem rekombinanten heterologen DNA-Konstrukt verliehen wird, das ein Gen umfasst, welches ein Hap3-Protein codiert, das eine mit der Consensus-Aminosäuresequenz von SEQ ID Nr. 8 oder SEQ ID Nr. 9 identische Aminosäuresequenz aufweist; und
(b) heterologe rekombinante DNA, die ein Herbizidresistenzmerkmal und ein Schädlingsresistenzmerkmal verleiht.

2. Maissamen gemäß Anspruch 1, wobei der Hybridmaissamen eine Resistenz gegen wenigstens ein Herbizid aufweist, das aus einem Glyphosat-Herbizid, einem Phosphinothricin-Herbizid, einem Oxynil-Herbizid, einem Imidazolinon-Herbizid, einem Dinitroanilin-Herbizid, einem Pyridin-Herbizid, einem Sulfonylharnstoff-Herbizid, einem Bialaphos-Herbizid, einem Sulfonamid-Herbizid und einem Gluphosinat-Herbizid ausgewählt ist.

3. Maissamen gemäß Anspruch 1, wobei der Hybridmaissamen eine Resistenz gegen wenigstens zwei Herbizide aufweist, die aus einem Glyphosat-Herbizid, einem Phosphinothricin-Herbizid, einem Oxynil-Herbizid, einem Imidazolinon-Herbizid, einem Dinitroanilin-Herbizid, einem Pyridin-Herbizid, einem Sulfonylharnstoff-Herbizid, einem Bialaphos-Herbizid, einem Sulfonamid-Herbizid und einem Gluphosinat-Herbizid ausgewählt sind.

4. Pflanze, die rekombinante DNA aufweist, welche sowohl ein Kulturpflanzen-Verbesserungsmerkmal als auch ein Herbizid- und/oder Schädlingsresistenzmerkmal verleiht, wobei es sich bei dem Kulturpflanzen-Verbesserungsmerkmal um Wassermangeltoleranz handelt, die von einem rekombinanten heterologen DNA-Konstrukt verliehen wird, das ein Gen umfasst, welches ein Hap3-Protein codiert, das eine mit der ConsensusAminosäuresequenz von SEQ ID Nr. 8 identische Aminosäuresequenz aufweist und/oder wenigstens eine der terminalen Aminosäure-Consensussequenzen von SEQ ID Nr. 9 oder 10 aufweist.

5. Pflanze gemäß Anspruch 4, wobei die Pflanze eine Mais-, Weizen-, Sonnenblumen-, Sorghum-, Luzerne-, Gerste-, Hirse-, Reis-, Tabak-, Obst- oder Gemüsepflanze ist.

6. Pflanze gemäß Anspruch 4 oder 5, die eine terminale Aminosäuresequenz aufweist, die mit der Consensus-Aminosäuresequenz von SEQ ID Nr. 10 identisch ist.

## Revendications

1. Graine de maïs hybride ayant dans son génome :
(a) un ADN recombinant hétérologue qui confère un caractère d'amélioration de cultures et une résistance aux herbicides, où ledit caractère d'amélioration de cultures est une tolérance à un déficit en eau conférée par une construction d'ADN hétérologue recombinant comprenant un gène qui code pour une protéine Hap3 ayant une séquence d'acides aminés identique à la séquence consensus d'acides aminés de la séquence SEQ ID NO : 8 ou SEQ ID NO : 9, et
(b) un ADN recombinant hétérologue qui confère un caractère de résistance aux herbicides et un caractère de résistance aux ravageurs.

2. Graine de maïs de la revendication 1, dans laquelle la graine de maïs hybride a une résistance à au moins un herbicide choisi parmi un herbicide glyphosate, un herbicide phosphinothricine, un herbicide oxynil, un herbicide imidazolinone, un herbicide dinitroaniline, un herbicide pyridine, un herbicide sulfonylurée, un herbicide bialaphos, un herbicide sulfonamide et un herbicide glufosinate.

3. Graine de maïs de la revendication 1, dans laquelle la graine de maïs hybride a une résistance à au moins deux herbicides choisis parmi un herbicide glyphosate, un herbicide phosphinothricine, un herbicide oxynil, un herbicide imidazolinone, un herbicide dinitroaniline, un herbicide pyridine, un herbicide sulfonylurée, un herbicide bialaphos, un herbicide sulfonamide et un herbicide glufosinate.

4. Plante ayant un ADN recombinant qui confère à la fois un caractère d'amélioration de cultures et un caractère de résistance aux herbicides et/ou aux ravageurs, où ledit caractère d'amélioration de cultures est une tolérance à un déficit en eau conférée par une construction d'ADN hétérologue recombinant comprenant un gène qui code pour une protéine Hap3 ayant une séquence d'acides aminés identique à la séquence consensus d'acides aminés de la séquence SEQ ID NO : 8 et/ou ayant au moins l'une des séquences consensus d'acides aminés terminales de la séquence SEQ ID NO : 9 ou 10.

5. Plante de la revendication 4, dans laquelle la plante est le maïs, le blé, le tournesol, le sorgho, la luzerne, l'orge, le millet, le riz, le tabac, un fruit ou une plante légumière.

6. Plante de la revendication 4 ou 5, qui a une séquence d'acides aminés terminale identique à une séquence consensus d'acides aminés de la séquence SEQ ID NO : 10.


### SEQUENCE LISTING

<110> Monsanto Technology, LLC
<120> Stacking Crop Improvement Traits in Transgenic Plants
<130> 090001ep
<140> EP10175642.7
   <141> 2004-10-01
<150> EP04793919.4
   <151> 2004-10-01
<150> PCT/US04/032191
   <151> 2004-10-01
<150> US 60/508409
   <151> 2003-10-02
<160> 10
<170> PatentIn version 3.2
<210> 1
   <211> 2480
   <212> DNA
   <213> Artificial
<220>
   <223> transcriptional unit comprising promoter, coding sequence for transcription factor of SEQ ID NO:2 and terminator elements
<400> 1
   aggatattaa agtatgtatt catcattaat ataatcagtg tattccaata tgtactacga    60
   tttccaatgt ctttattgtc gccgtatgta atcggcgtca caaaataatc cccggtgact   120
   ttcttttaat ccaggatgaa ataatatgtt attataattt ttgcgatttg gtccgttata   180
   ggaattgaag tgtgcttgag ctcggtcgcc accactccca tttcataatt ttacatgtat   240
   ttgaaaaata aaaatttatg gtattcaatt taaacacgta tacttgtaaa gaatgatatc   300
   ttgaaagaaa tatagtttaa atatttattg ataaaataac aagtcaggta ttatagtcca   360
   agcaaaaaca taaatttatt gatgcaagtt taaattcaga aatatttcaa taactgatta   420
   tatcagctgg tacattgccg tagatgaaag actgagtgcg atattatgtg taatacataa   480
   attgatgata tagctagaac tagtggatcc cccgggccct gcaggctcga gctagtttga   540
   gatatccccg ttatggtact ggggttgcat ataacccatt ccttggttgt atgctccctg   600
   ttggcccatc ccttgtgcag ctgagctact tgctcccaca tgaccaaggg catccttttt   660
   aattgagcca tcgctagatt ttgcagttaa cttgctatca ccctccatct ctctgtactt   720
   ctgcaggtac accttgaggg gttcaatgta gtcttcaaac cccagcgtgg ccatggccca   780
   cagcagatcg tcgccattga tggtcttccg cttctccctc tggcacttgt cactcgcttc   840
   gctagtgatg aaggagatga actcggagac gcactcctgc acggtctcct tagcgtcctt   900
   ggcgatcttc ccgttagccg ggatggtctt cccgttagcc gggatggcct tcttcatgat 960
   gcgactgatg ttggcgatgg gcaggaacct gtcctgctcc ctgacgctgc caccgcctcc 1020
   gcctcccctg gggctcccgc tctcgtggct cccgccgccg ccgccagggc tcgccggagc 1080
   ttccgccatg gtctacctac aaaaaagctc cgcacgaggc tgcatttgtc acaaatcatg 1140
   aaaagaaaaa ctaccgatga acaatgctga gggattcaaa ttctacccac aaaaagaaga 1200
   aagaaagatc tagcacatct aagcctgacg aagcagcaga aatatataaa aatataaacc 1260
   atagtgccct tttcccctct tcctgatctt gtttagcatg gcggaaattt taaacccccc 1320
   atcatctccc ccaacaacgg cggatcgcag atctacatcc gagagcccca ttccccgcga 1380
   gatccgggcc ggatccacgc cggcgagagc cccagccgcg agatcccgcc cctcccgcgc 1440
   accgatctgg gcgcgcacga agccgcctct cgcccaccca aactaccaag gccaaagatc 1500
   gagaccgaga cggaaaaaaa aaacggagaa agaaagagga gaggggcggg gtggttaccg 1560
   gcggcggcgg aggggagggg gggaggagct cgtcgtccgg cagcgagggg ggaggaggtg 1620
   gtggtggtgg tggtggtagg gttgggggga tgggaggaga ggggggggta tgtatatagt 1680
   ggcgatgggg ggcgtttctt tggaagcgga gggagggccg gcctcgtcgc tggctcgcga 1740
   tcctcctcgc gtttccggcc cccacgaccc ggacccacct gctgtttttt ctttttcttt 1800
   tttttctttc tttttttttt tttggctgcg agacgtgcgg tgcgtgcgga caactcacgg 1860
   tgatagtggg ggggtgtgga gactattgtc cagttggctg gactggggtg ggttgggttg 1920
   ggttgggttg ggctgggctt gctatggatc gtggatagca ctttgggctt taggacttta 1980
   ggggttgttt ttgtaaatgt tttgagtcta agtttatctt ttatttttac tagaaaaaat 2040
   acccatgcgc tgcaacgggg gaaagctatt ttaatcttat tattgttcat tgtgagaatt 2100
   cgcctgaata tatatttttc tcaaaaatta tgtcaaatta gcatatgggt ttttttaaag 2160
   atatttctta tacaaatccc tctgtattta caaaagcaaa cgaacttaaa acccgactca 2220
   aatacagata tgcatttcca aaagcgaata aacttaaaaa ccaattcata caaaaatgac 2280
   gtatcaaagt accgacaaaa acatcctcaa tttttataat agtagaaaag agtaaatttc 2340
   actttgggcc accttttatt accgatattt tactttatac caccttttaa ctgatgtttt 2400
   cacttttgac caggtaatct tacctttgtt ttattttgga ctatcccgac tctcttctca 2460
   agcatatgaa tgacctcgag 2480
<210> 2
   <211> 185
   <212> PRT
   <213> Zea mays
<400> 2
   Met Ala Glu Ala Pro Ala Ser Pro Gly Gly Gly Gly Gly Ser His Glu
   1               5                   10                  15
   Ser Gly Ser Pro Arg Gly Gly Gly Gly Gly Gly Ser Val Arg Glu Gln
               20                  25                  30
   Asp Arg Phe Leu Pro Ile Ala Asn Ile Ser Arg Ile Met Lys Lys Ala
           35                  40                  45
   Ile Pro Ala Asn Gly Lys Thr Ile Pro Ala Asn Gly Lys Ile Ala Lys
       50                  55                  60
   Asp Ala Lys Glu Thr Val Gln Glu Cys Val Ser Glu Phe Ile Ser Phe
   65                  70                  75                  80
   Ile Thr Ser Glu Ala Ser Asp Lys Cys Gln Arg Glu Lys Arg Lys Thr
                   85                  90                  95
   Ile Asn Gly Asp Asp Leu Leu Trp Ala Met Ala Thr Leu Gly Phe Glu
               100                 105                 110
   Asp Tyr Ile Glu Pro Leu Lys Val Tyr Leu Gln Lys Tyr Arg Glu Met
           115                 120                 125
   Glu Gly Asp Ser Lys Leu Thr Ala Lys Ser Ser Asp Gly Ser Ile Lys
       130                 135                 140
   Lys Asp Ala Leu Gly His Val Gly Ala Ser Ser Ser Ala Ala Gln Gly
   145                 150                 155                 160
   Met Gly Gln Gln Gly Ala Tyr Asn Gln Gly Met Gly Tyr Met Gln Pro
                   165                 170                 175
   Gln Tyr His Asn Gly Asp Ile Ser Asn
               180                 185
<210> 3
   <211> 178
   <212> PRT
   <213> Zea mays
<400> 3
   Met Ala Glu Ala Pro Ala Ser Pro Gly Gly Gly Gly Gly Ser His Glu
   1               5                   10                  15
   Ser Gly Ser Pro Arg Gly Gly Gly Gly Gly Gly Ser Val Arg Glu Gln
               20                  25                  30
   Asp Arg Phe Leu Pro Ile Ala Asn Ile Ser Arg Ile Met Lys Lys Ala
           35                  40                  45
   Ile Pro Ala Asn Gly Lys Ile Ala Lys Asp Ala Lys Glu Thr Val Gln
       50                  55                  60
   Glu Cys Val Ser Glu Phe Ile Ser Phe Ile Thr Ser Glu Ala Ser Asp
   65                  70                  75                  80
   Lys Cys Gln Arg Glu Lys Arg Lys Thr Ile Asn Gly Asp Asp Leu Leu
                   85                  90                  95
   Trp Ala Met Ala Thr Leu Gly Phe Glu Asp Tyr Ile Glu Pro Leu Lys
               100                 105                 110
   Val Tyr Leu Gln Lys Tyr Arg Glu Met Glu Gly Asp Ser Lys Leu Thr
           115                 120                 125
   Ala Lys Ser Ser Asp Gly Ser Ile Lys Lys Asp Ala Leu Gly His Val
       130                 135                 140
   Gly Ala Ser Ser Ser Ala Ala Glu Gly Met Gly Gln Gln Gly Ala Tyr
   145                 150                 155                 160
   Asn Gln Gly Met Gly Tyr Met Gln Pro Gln Tyr His Asn Gly Asp Ile
                   165                 170                 175
   Ser Asn
<210> 4
   <211> 537
   <212> DNA
   <213> Zea mays
<400> 4
   atggcggaag ctccggcgag ccctggcggc ggcggcggga gccacgagag cgggagcccc      60
   aggggaggcg gaggcggtgg cagcgtcagg gagcaggaca ggttcctgcc catcgccaac     120
   atcagtcgca tcatgaagaa ggccatcccg gctaacggga agatcgccaa ggacgctaag     180
   gagaccgtgc aggagtgcgt ctccgagttc atctccttca tcactagcga agcgagtgac     240
   aagtgccaga gggagaagcg gaagaccatc aatggcgacg atctgctgtg ggccatggcc     300
   acgctggggt ttgaagacta cattgaaccc ctcaaggtgt acctacagaa gtacagagag     360
   atggagggtg atagcaagtt aactgctaaa tctagcgatg gctcgattaa aaaggatgct     420
   cttggtcatg tgggagcaag tagctcagct gcagaaggga tgggccaaca gggagcatac     480
   aaccaaggaa tgggttatat gcaacctcag taccataacg gggatatctc aaactaa        537
<210> 5
   <211> 522
   <212> DNA
   <213> Glycine max
<400> 5
   atgtcggatg cgccaccgag cccgactcat gagagtgggg gcgagcagag cccgcgcggt      60
   tcgtcgtccg gcgcgaggga gcaggaccgg tacctcccga ttgccaacat cagccgcatt     120
   atgaagaagg ctctgcctcc caacggcaag attgcaaagg atgccaaaga caccatgcag     180
   gaatgcgttt ctgagttcat cagcttcatt accagcgagg cgagtgagaa atgccagaag     240
   gagaagagaa agacaatcaa tggagacgat ttgctatggg ccatggccac tttaggattt     300
   gaagactaca tagagccgct taaggtgtac ctggctaggt acagagaggc ggagggtgac     360
   actaaaggat ctgctagaag tggtgatgga tctgctacac cagatcaagt tggccttgca     420
   ggtcaaaatt ctcagcttgt tcatcagggt tcgctgaact atattggttt gcaggtgcaa     480
   ccacaacatc tggttatgcc ttcaatgcaa agccatgaat ag                        522
<210> 6
   <211> 173
   <212> PRT
   <213> Glycine max
<400> 6
   Met Ser Asp Ala Pro Pro Ser Pro Thr His Glu Ser Gly Gly Glu Gln
   1 5 10 15
   Ser Pro Arg Gly Ser Ser Ser Gly Ala Arg Glu Gln Asp Arg Tyr Leu
   20 25 30
   Pro Ile Ala Asn Ile Ser Arg Ile Met Lys Lys Ala Leu Pro Pro Asn
   35 40 45
   Gly Lys Ile Ala Lys Asp Ala Lys Asp Thr Met Gln Glu Cys Val Ser
   50 55 60
   Glu Phe Ile Ser Phe Ile Thr Ser Glu Ala Ser Glu Lys Cys Gln Lys
   65 70 75 80
   Glu Lys Arg Lys Thr Ile Asn Gly Asp Asp Leu Leu Trp Ala Met Ala
   85 90 95
   Thr Leu Gly Phe Glu Asp Tyr Ile Glu Pro Leu Lys Val Tyr Leu Ala
   100 105 110
   Arg Tyr Arg Glu Ala Glu Gly Asp Thr Lys Gly Ser Ala Arg Ser Gly
   115 120 125
   Asp Gly Ser Ala Thr Pro Asp Gln Val Gly Leu Ala Gly Gln Asn Ser
   130 135 140
   Gln Leu Val His Gln Gly Ser Leu Asn Tyr Ile Gly Leu Gln Val Gln
   145 150 155 160
   Pro Gln His Leu Val Met Pro Ser Met Gln Ser His Glu
   165 170
<210> 7
   <211> 141
   <212> PRT
   <213> Arabidopsis thaliana
<400> 7
   Met Ala Asp Thr Pro Ser Ser Pro Ala Gly Asp Gly Gly Glu Ser Gly
   1               5                   10                  15
   Gly Ser Val Arg Glu Gln Asp Arg Tyr Leu Pro Ile Ala Asn Ile Ser
               20                  25                  30
   Arg Ile Met Lys Lys Ala Leu Pro Pro Asn Gly Lys Ile Gly Lys Asp
           35                  40                  45
   Ala Lys Asp Thr Val Gln Glu Cys Val Ser Glu Phe Ile Ser Phe Ile
       50                  55                  60
   Thr Ser Glu Ala Ser Asp Lys Cys Gln Lys Glu Lys Arg Lys Thr Val
   65                  70                  75                  80
   Asn Gly Asp Asp Leu Leu Trp Ala Met Ala Thr Leu Gly Phe Glu Asp
                   85                  90                  95
   Tyr Leu Glu Pro Leu Lys Ile Tyr Leu Ala Arg Tyr Arg Glu Leu Glu
               100                 105                 110
   Gly Asp Asn Lys Gly Ser Gly Lys Ser Gly Asp Gly Ser Asn Arg Asp
           115                 120                 125
   Ala Gly Gly Gly Val Ser Gly Glu Glu Met Pro Ser Trp
       130                 135                 140
<210> 8
   <211> 101
   <212> PRT
   <213> Artificial sequence
<220>
   <223> protein consensus sequence
<220>
   <221> MISC_FEATURE
   <222> (6)..(6)
   <223> Xaa can be Tyr or Phe
<220>
   <221> MISC_FEATURE
   <222> (20)..(20)
   <223> Xaa can be Leu or Ile
<220>
   <221> MISC_FEATURE
   <222> (22)..(22)
   <223> Xaa can be Ala or Pro
<220>
   <221> MISC_FEATURE
   <222> (26)..(26)
   <223> Xaa can be Thr or none
<220>
   <221> MISC_FEATURE
   <222> (27)..(27)
   <223> Xaa can be Ile or none
<220>
   <221> MISC_FEATURE
   <222> (28)..(28)
   <223> Xaa can be Pro or none
<220>
   <221> MISC_FEATURE
   <222> (29).. (29)
   <223> Xaa can be Ala or none
<220>
   <221> MISC_FEATURE
   <222> (30)..(30)
   <223> Xaa can be Asn or none
<220>
   <221> MISC_FEATURE
   <222> (31)..(31)
   <223> Xaa can be Gly or none
<220>
   <221> MISC_FEATURE
   <222> (32)..(32)
   <223> Xaa can be Lys or none
<220>
   <221> MISC_FEATURE
   <222> (34)..(34)
   <223> Xaa can be Ala or Gly
<220>
   <221> MISC_FEATURE
   <222> (39).. (39)
   <223> Xaa can be Glu or Asp
<220>
   <221> MISC_FEATURE
   <222> (41)..(41)
   <223> Xaa can be Val or Met
<220>
   <221> MISC_FEATURE
   <222> (58)..(58)
   <223> Xaa can be Asp or Glu
<220>
   <221> MISC_FEATURE
   <222> (62)..(62)
   <223> Xaa can be Arg or Lys
<220>
   <221> MISC_FEATURE
   <222> (68)..(68)
   <223> Xaa can be Ile or Val
<220>
   <221> MISC_FEATURE
   <222> (86)..(86)
   <223> Xaa can be Leu or Ile
<220>
   <221> MISC_FEATURE
   <222> (91)..(91)
   <223> Xaa can be Val or Ile
<220>
   <221> MISC_FEATURE
   <222> (94)..(94)
   <223> Xaa can be Gln or Ala
<220>
   <221> MISC_FEATURE
   <222> (95)..(95)
   <223> Xaa can be Lys or Arg
<220>
   <221> MISC_FEATURE
   <222> (99)..(99)
   <223> Xaa can be Met or Ala or Leu
<400> 8
   Arg Glu Gln Asp Arg Xaa Leu Pro Ile Ala Asn Ile Ser Arg Ile Met
   1               5                   10                  15
   Lys Lys Ala Xaa Pro Xaa Asn Gly Lys Xaa Xaa Xaa Xaa Xaa Xaa Xaa
               20                  25                  30
   Ile Xaa Lys Asp Ala Lys Xaa Thr Xaa Gln Glu Cys Val Ser Glu Phe
           35                  40                  45
   Ile Ser Phe Ile Thr Ser Glu Ala Ser Xaa Lys Cys Gln Xaa Glu Lys
       50                  55                  60
   Arg Lys Thr Xaa Asn Gly Asp Asp Leu Leu Trp Ala Met Ala Thr Leu
   65                  70                  75                  80
   Gly Phe Glu Asp Tyr Xaa Glu Pro Leu Lys Xaa Tyr Leu Xaa Xaa Tyr
                   85                  90                  95
   Arg Glu Xaa Glu Gly
               100
<210> 9
   <211> 55
   <212> PRT
   <213> Artificial
<220>
   <223> consensus protein sequence
<220>
   <221> MISC_FEATURE
   <222> (29)..(29)
   <223> Xaa can be Gln or Glu
<400> 9
   Asp Ser Lys Leu Thr Ala Lys Ser Ser Asp Gly Ser Ile Lys Lys Asp
   1               5                   10                  15
   Ala Leu Gly His Val Gly Ala Ser Ser Ser Ala Ala Xaa Gly Met Gly
               20                  25                  30
   Gln Gln Gly Ala Tyr Asn Gln Gly Met Gly Tyr Met Gln Pro Gln Tyr
           35                  40                  45
   His Asn Gly Asp Ile Ser Asn
       50                  55
<210> 10
   <211> 8
   <212> PRT
   <213> Artificial
<220>
   <223> consensus protein sequence
<220>
   <221> MISC_FEATURE
   <222> (2)..(4)
   <223> Xaa can be any naturally occuring amino acid
<220>
   <221> MISC_FEATURE
   <222> (6)..(6)
   <223> Xaa can be any naturally occuring amino acid
<400> 10
   Met Xaa Xaa Xaa Pro Xaa Ser Pro
   1               5
   1